# EUROPEAN PATENT SPECIFICATION

(11) **EP 1 750 664 B1**
(45) Date of publication and mention of the grant of the patent: **15.07.2009**
(21) Application number: 05738844.9
(22) Date of filing: 27.04.2005
(51) Int. Cl.: A61K 9/00, A61K 31/00, A61P 27/02, A61K 41/00

(54) **INTRAVITREAL IMPLANTS IN CONJUNCTION WITH PHOTODYNAMIC THERAPY TO IMPROVE VISION**
INTRAVITREALE IMPLANTATE IN VERBINDUNG MIT PHOTODYNAMISCHER THERAPIE ZUR VERBESSERUNG DER SEHKRAFT
IMPLANTS INTRAVITREENS REALISES CONJOINTEMENT AVEC UNE THERAPIE PHOTODYNAMIQUE AFIN D'AMELIORER LA VISION

(30) Priority: 30.04.2004 US 837348; 22.04.2005 US 112523
(43) Date of publication of application: 14.02.2007
(73) Proprietor: ALLERGAN, INC., Irvine CA 92612 (US)
(72) Inventor: WHITCUP, Scott, M., Laguna Hills, CA 92653 (US); BURKE, James, A., Santa Ana, CA 92705 (US); WEINBERG, David, V., Whitefish Bay, WI 53217 (US)
(74) Representative: HOFFMANN EITLE
(86) International application number: PCT/US2005/014201
(87) International publication number: WO 2005/110365

(56) References cited:
- US-A1- 2003 083 649
- SPAIDE RICHARD F ET AL: "Combined photodynamic therapy with verteporfin and intravitreal triamcinolone acetonide for choroidal neovascularization." OPHTHALMOLOGY. AUG 2003, vol. 110, no. 8, August 2003 (2003-08), pages 1517-1525, XP002338289 ISSN: 0161-6420
- RECHTMAN E ET AL: "Intravitreal triamcinolone with photodynamic therapy for subfoveal choroidal neovascularisation in age related macular degeneration." THE BRITISH JOURNAL OF OPHTHALMOLOGY. MAR 2004, vol. 88, no. 3, March 2004 (2004-03), pages 344-347, XP002338290 ISSN: 0007-1161
- SPAIDE R F ET AL: "COMBINED PHOTODYNAMIC THERAPY WITH VERTEPORFIN AND INTRAVITREAL TRIAMCINOLONE ACETONIDE FOR CHOROIDAL NEOVASCULARIZATION." ARVO ANNUAL MEETING ABSTRACT SEARCH AND PROGRAM PLANNER, vol. 2003, 2003, page Abstract No. 5032, XP009051457 & ANNUAL MEETING OF THE ASSOCIATION FOR RESEARCH IN VISION AND OPHTHALMOLOGY; FORT LAUDERDALE, FL, USA; MAY 04-08, 2003
- SPEE C K ET AL: "IMPACT OF TRIAMCINOLONE ON HIGH DOSES OF PDT IN THE RABBIT CHORIORETINA" ARVO ANNUAL MEETING ABSTRACT SEARCH AND PROGRAM PLANNER, vol. 2003, 2003, page Abstract No. 5006, XP009051456 & ANNUAL MEETING OF THE ASSOCIATION FOR RESEARCH IN VISION AND OPHTHALMOLOGY; FORT LAUDERDALE, FL, USA; MAY 04-08, 2003
- LI R ET AL: "EFFECTS OF TRIAMCINOLONE DOSE AND TIMING ON PHOTODYNAMIC THERAPY WITH VERTEPORFIN IN THE RABBIT CHORIORETINA" ARVO ANNUAL MEETING ABSTRACT SEARCH AND PROGRAM PLANNER, vol. 2003, 2003, page Abstract No. 5005, XP009051455 & ANNUAL MEETING OF THE ASSOCIATION FOR RESEARCH IN VISION AND OPHTHALMOLOGY; FORT LAUDERDALE, FL, USA; MAY 04-08, 2003
- RECHTMAN E ET AL: "THE SAFETY AND EFFICACY OF PHOTODYNAMIC THERAPY WITH VERTEPORFIN COMBINED WITH INTRAVITREAL TRIAMCINOLONE ACETONIDE INJECTIONS FOR THE TREATMENT OF NEW SUBFOVEAL CHOROIDAL NEOVASCULARIZATION DUE TO AGE - RELATED MACULAR DEGENERATION." ARVO ANNUAL MEETING ABSTRACT SEARCH AND PROGRAM PLANNER, vol. 2003, 2003, page Abstract No. 1742, XP009051454 & ANNUAL MEETING OF THE ASSOCIATION FOR RESEARCH IN VISION AND OPHTHALMOLOGY; FORT LAUDERDALE, FL, USA; MAY 04-08, 2003
- HUNT D W C ET AL: "STATUS OF THERAPIES IN DEVELOPMENT FOR THE TREATMENT OF AGE-RELATED MACULAR DEGENERATION" IDRUGS, CURRENT DRUGS LTD, GB, vol. 6, no. 5, May 2003 (2003-05), pages 464-469, XP008041783 ISSN: 1369-7056

## Description

### BACKGROUND

The present invention generally relates to the use of a combination as described is claim 1 for treating eyes, and more specifically for treating eyes using photodynamic therapy in conjunction with intraocular implants.

Loss of visual acuity is a common problem associated with aging and with various conditions of the eye. Particularly troublesome is the development of unwanted neovascularization in the cornea, retina or choroid. Choroidal neovascularization (CNV) involves abnormal growth of blood vessels from the choroid through Bruch's membrane to the region beneath the retina. The abnormal blood growth results in leakage and bleeding into the subretinal space, which may result in scar formation beneath the macula of the retina and a loss of vision. CNV leads to hemorrhage and fibrosis, with resultant visual loss in a number of recognized eye diseases, including macular degeneration, ocular histoplasmosis syndrome, myopia, diabetic retinopathy and inflammatory diseases.

Macular degeneration, such as age related macular degeneration ("AMD") is the leading cause of blindness in the world. It is estimated that thirteen million Americans have evidence of macular degeneration. Macular degeneration results in a break down the macula, the light-sensitive part of the retina responsible for the sharp, direct vision needed to read or drive. Central vision is especially affected. Macular degeneration is diagnosed as either dry (atrophic) or wet (exudative). The dry form of macular degeneration is more common than the wet form of macular degeneration, with about 90% of AMD patients being diagnosed with dry AMD. The wet form of the disease usually leads to more serious vision loss. Macular degeneration can produce a slow or sudden painless loss of vision. The cause of macular degeneration is not clear. The dry form of AMD may result from the aging and thinning of macular tissues, depositing of pigment in the macula, or a combination of the two processes. With wet AMD, new blood vessels grow beneath the retina and leak blood and fluid. This leakage causes retinal cells to die and creates blind spots in central vision. Current treatments for macular degeneration are generally limited to those aimed at preventing further progression of the disease.

Macular edema ("ME") is a swelling of the macula. The edema is caused by fluid leaking from retinal blood vessels. Blood leaks out of the weak vessel walls into a very small area of the macula which is rich in cones, the nerve endings that detect color and from which daytime vision depends. Blurring then occurs in the middle or just to the side of the central visual field. Visual loss can progress over a period of months. Diabetes, retinal blood vessel obstruction, eye inflammation, and age-related macular degeneration have all been associated with macular edema. The macula may also be affected by swelling following cataract extraction. Current treatment for ME includes topical anti-inflammatory drops. In some cases, medication is injected near the back of the eye for a more concentrated effect. Oral medications are also sometimes prescribed.

Traditionally, CNV has been treated by occluding the abnormal blood vessels with thermal energy transmitted from a laser. Thermal photocoagulation of the blood vessels undesirably results in full-thickness retinal damage, as well as damage to medium and large choroidal blood vessels. More recently, lasers have been used to provide more selective closure or occlusion of the abnormal blood vessels. One example includes the use of photosensitive chemical compounds that are activated by electromagnetic energy transmitted from a laser; this treatment is commonly referred to as photodynamic therapy. With photodynamic therapy, a patient typically receives an injection of a photoactive compound. The photoactive compound accumulates within the CNV at which point a laser is used to direct relatively low power electromagnetic energy of a specified wavelength particular for the photoactive compound. Using a low power laser reduces the potential of thermal damage associated with traditional techniques. When the photoactive compound is activated by absorbing the energy from the laser, reactive ion species, such as free radicals, are generated which cause cellular destruction and result in occlusion of the CNV.

Diabetic retinopathy is characterized by angiogenesis. Small blood vessels on the retina of the eye are damaged, resulting in the growth of abnormal blood vessels which proliferate and eventually leak and blur or otherwise obscure vision. Laser surgery is the current mainstay of treatment for diabetic retinopathy. Advanced proliferative diabetic retinopathy may be treated by vitrectomy, which includes removal of a portion of the vitreous and replacement with a clear replacement material. In any event, early treatment of diabetic retinopathy is essential to preventing permanent vision loss.

Glaucoma is a serious ocular condition characterized by increased ocular pressure and loss of retinal ganglion cells. Damage caused by glaucoma is thought to be irreversible. Current treatments for early stage glaucoma usually involve therapeutic eyedrops and oral medications used to lower ocular pressure.

Uveitis involves inflammation of structures of the uvea. Treatment may consist of topical eyedrops or ointments containing corticosteroids.

Retinitis pigmentosa is characterized by retinal degeneration. Retinitis pigmentosa is considered to be not one disease, but rather a group of diseases with common attributes. Visual problems common to retinitis pigmentosa include tunnel vision field, night blindness, glare problems, double vision and development of cataracts. Currently, there are no standard treatments available for retinitis pigmentosa, though it is believed that increasing intake of Vitamin A may slow progression of the disease.

What is needed then are more effective methods for treating ocular conditions. The present invention is concerned with and directed to methods for treatment of these and other ocular conditions.

The following patents and additional publications include disclosure which is relevant to and/or helpful in understanding the present invention.

Weber et al., U.S. Patent Application Serial No. 10/246,884, filed on September 18, 2002, having Pub. No. US 2004/0054374 A1, describes apparatus and methods for delivering ocular implants into an eye of a patient.

Biocompatible implants for placement in the eye have been disclosed in a number of patents, such as U.S. Pat. Nos. 4,521,210; 4,853,224; 4,997,652; 5,164,188; 5,443,505; 5,501,856; 5,766,242; 5,824,072; 5,869,079; 6,074,661; 6,331,313; 6,369,116; and 6,699,493.

Zhou et al. discloses a multiple-drug implant comprising 5-fluorouridine, triamcinolone, and human recombinant tissue plasminogen activator for intraocular management of proliferative vitreoretinopathy . Zhou, T., et al. "Development of a multiple-drug delivery implant for intraocular management of proliferative vitreoretinopathy", Journal of Controlled Release 55: pp. 281-295.

Heller, "Biodegradable Polymers in Controlled Drug Delivery", in: CRC Critical Reviews in Therapeutic Drug Carrier Systems, Vol. 1, (CRC Press, Boca Raton, FL, 1987), pp 39-90, describes encapsulation for controlled drug delivery. Heller, in: Hydrogels in Medicine and Pharmacy, N. A. Peppes ed., Vol. III, (CRC Press, Boca Raton, FL, 1987), pp 137-149, describes bioerodible polymers.

Anderson et al., Contraception 13:375, (1976), and; Miller et al., J. Biomed. Materials Res. 11:711, (1977) describe various properties of poly(dL-lactic acid).

Brine, U.S. Pat. No. 5,075,115 discloses controlled release formulations with lactic acid polymers and co-polymers.

Di Colo, Biomaterials 13:850-856 (1992) describes controlled drug release from hydrophobic polymers.

Other documents that are also relevant or otherwise helpful in understanding the present invention are Mori et al. U.S. Patent No. RE37,180, Bommer et al. U.S. Patent 4,656,186, Bommer et al. U.S. Patent 4,675,338, Bommer et al. U.S. Patent 4,693,885, Dougherty et al. U.S. Patent 4,932,934, Pandey et al. U.S. Patent 5,198,460, Pandey et al. U.S. Patent 5,314,905, Pandey et al. U.S. Patent 5,459,159, Pelka et al. U.S. Patent 5,655,832, Traunder et al. U.S. Patent 5,913,884, Meyer et al. U.S. Patent 6,217,869, Hearst et al. U.S. Patent 6,258,319, Sinofsky U.S. Patent 6,270,492, Blumenkranz et al. U.S. Patent 6,270,749, Richter et al. U.S. Patent 6,274,614, Russell U.S. Patent 6,290,713, Horowitz et al. U.S. Patent 6,294,361, Glossop U.S. Patent 6,317,616, Harth et al. U.S. Patent Application Publication US 2001/0023363 A1, U.S. Patent Application Publication US 2002/0040015A1, U.S. patent application serial number 10/020,541, U.S. patent application serial number 09/998,718, and Conquelet et al, "Successful Photodynamic Therapy Combined with Laser Photocoagulation in Three Eyes with Classic Subfoveal Choroidal Neovascularization Affecting Two Patients with Multifocal Choroiditis: Case Reports", Bull. Soc. Belge Ophtalmol, 283, 69-73, 2002.

R. F. Spaid et al. ("Ophthalmology", Volume 110, no. 8, August 2003, pages 1517-1525) and E. Rechtman et al. ("The British Journal of Ophthalmology", Volume 88, no. 3, March 2004, pages 344-347) disclose the use of a combination of photodynamic therapy using verteporfin and intravitreal injection of triamcinolone acetonide for treating choroidal neovascularization secondary to age-related macular degeneration.

### SUMMARY

The present invention provides the use of a combination therapy as described in claim 1 for treating conditions of an eye, for example, to achieve one or more desired therapeutic effects.

In a broad aspect of the invention, a use of combination therapy for treating an eye is provided, wherein the therapy comprises the steps of placing into an eye, a bioerodible implant comprising an anti-inflammatory component and a bioerodible polymeric component, introducing a photoactive agent into the eye, and irradiating the eye with electromagnetic radiation, for example light energy, in order to activate the photoactive agent in the eye.

The present invention is especially effective for treating conditions of the eye characterized, at least in part, by retinal abnormalities, for example choroidal neovascularization (CNV).

The anti-inflammatory component of the implant may comprise one or more anti-inflammatory agents, and preferably comprises a steroidal anti-inflammatory agent or a non-steroidal anti-inflammatory agent. In some embodiments of the invention, the anti-inflammatory component comprises a therapeutically active agent selected from the group consisting of cortisone, dexamethasone, fluocinolone, hydrocortisone, methylprednisolone, prednisolone, prednisone, and triamcinolone, derivatives thereof and mixtures thereof. The anti-inflammatory agent may be selected from the group consisting of corticosteroids and mixtures thereof. In certain particular implants, the anti-inflammatory component consists essentially of dexamethasone.

In some embodiments of the invention, the bioerodible implant comprises an ophthalmically acceptable therapeutic agent in addition to the anti-inflammatory component. For example, the implant may include, in addition to the anti-inflammatory agent, an antiviral agent, an antibiotic agent, an antifungal agent, an anti-cancer agent, an antiglaucoma agent, an analgesic, an immunomodulatory agent, a macro-molecule, or mixtures thereof.

In accordance with the disclosure herein, the implant is structured such that the anti-inflammatory agent is associated with, for example is dispersed within, the bioerodible polymeric component. For example, an implant used in a method of the invention can be formulated with particles of an active agent dispersed within a biodegradable polymer matrix. Release of the active agent can be achieved by erosion of the biodegradable polymer matrix and by diffusion of the particulate agent into an ocular fluid, for example, vitreal fluid, with contemporaneous or subsequent dissolution of the polymer matrix. Release of the active agent may be controlled based in part on a level of access of ocular fluid to the particulate agent through openings or pores of the implant. Additionally, implants may be used which include a non-biodegradable polymeric coating with one or more openings or orifices, such as the implants disclosed in U.S. Pat. No. 6,331,313.

The implants may be structured such that the bioerodible polymer is in the form of a matrix material comprising at least about 10 percent, at least about 20 percent, at least about 30 percent, at least about 40 percent, at least about 50 percent, at least about 60 percent, at least about 70 percent, at least about 80 percent, or at least about 90 percent by weight of the implant.

The release kinetics of the implants that are useful in the methods of the present invention can be dependent in part on other factors, such as, for example, the surface area of the implant. A larger surface area exposes more of the implant composition to ocular fluid, causing faster erosion of the polymer matrix and faster dissolution of the active agent particles in the fluid. Therefore, the size and shape of the implant may also be used to control the rate of release, period of treatment, and active agent concentration at the site of implantation. At equal active agent loads, larger implants will deliver a proportionately larger dose, but depending on the surface to mass ratio, may possess a slower release rate.

Other factors which influence the release kinetics of active agent from the implant can include such characteristics as the size and shape of the implant, the size of the active agent particles, the solubility of the active agent, the ratio of active agent to polymer(s), the method of manufacture, the surface area exposed, and the erosion rate of the polymer(s). The release kinetics achieved by degradation or erosion of the element are different than that achieved through formulations which release active agents through polymer swelling, such as with crosslinked hydrogels. In that case, the active agent is not released through polymer erosion, but through polymer swelling and drug diffusion, which releases agent as liquid diffuses through the pathways exposed. It is also contemplated that the presence an/or activation of the photoactive agent that has been introduced into the eye in accordance with the present invention may influence the release kinetics of active agent from the implant.

It is additionally noted that the release rate of the active agent from implants used in the methods in accordance with the invention can in some embodiments depend at least in part on the mechanism of degradation of the polymeric component or components making up the biodegradable polymer matrix. For example, condensation polymers may be degraded by hydrolysis (among other mechanisms) and therefore any change in the composition of the implant that enhances water uptake by the implant will likely increase the rate of hydrolysis, thereby increasing the rate of polymer degradation and erosion, and thus increasing the rate of active agent release.

The implants used in accordance with the invention may be of any geometry including particles, sheets, patches, plaques, films, discs, fibers, rods, and the like, or may be of any size or shape compatible with the selected site of implantation, as long as the implants have the desired release kinetics and deliver an amount of anti-inflammatory agent and in some instances, one or more other active agents that will be therapeutic for the intended medical condition of the eye. An upper limit for the implant size will be determined by factors such as the desired release kinetics, toleration for the implant at the site of implantation, size limitations on insertion, and ease of handling. For example, the vitreous chamber is able to accommodate relatively large rod-shaped implants, generally having diameters of about 0.05 mm to 3 mm and a length of about 0.5 to about 10 mm. In one variation, the rods have diameters of about 0.1 mm to about 1 mm. In another variation, the rods have diameters of about 0.3 mm to about 0.75 mm. In yet a further variation, other implants having variable geometries but approximately similar volumes may also be used.

Biodegradable implants may include one or more biodegradable polymers to form the biodegradable polymer component. In certain embodiments of the present implants, the bioerodible polymeric component useful in the methods of the present invention, comprises a mixture of a first biodegradable polymer having terminal acid groups, and a second biodegradable polymer having terminal acid groups. For example, the first biodegradable polymer may comprise a poly (D,L-lactide-co-glycolide).and the second biodegradable polymer may comprise a poly (D,L-lactide).

In some embodiments of the invention, the bioerodible polymeric component of the implant used in the methods for treating an eye includes a polymeric material selected from the group consisting of a polymer of poly-lactic acid, a polymer of poly-glycolic acid, a copolymer of lactic acid and glycolic acid, and combinations thereof.

In a preferred embodiment of the invention, the implant comprises an anti-inflammatory steroid, preferably dexamethasone, dispersed within a PLGA polymeric matrix material.

The photoactive agent may comprise any biocompatible agent that is activatable, for example is sensitive, when exposed to a form of electromagnetic radiation, for example light, for example, laser radiation.

Preferably, in accordance with the present invention, the photoactive agent comprises a chemical compound that, when introduced into the bloodstream of the patient, accumulates within or near retinal cells of an eye, and when exposed to electromagnetic energy, for example laser irradiation, becomes activated thereby. The photoactive agent may be used both diagnostically, such as for identifying areas of neovascularization, and therapeutically, such as for causing coagulation or other tissue reaction when exposed to light energy. For example in some embodiments of the invention, the photoactive agent may be effective to form, one or more reactive ion species, such as free radicals, when the photoactive agent is exposed to particular wavebands or particular wavelengths of light. These reactive ion species are effective in destruction of unwanted neovascularization in the retina.

Examples of suitable photoactive agents for purposes of the present invention include, but are not limited to, porphyrins, hematoporphyrins, hematoporphyrin derivatives, pheophorbides, derivatives of pheophorbides, benzoporphyrins, benzoporphyrin derivatives, such as verteporfin, bacteriochlorins, purpurins, merocyanines, porphycenes, tricarbocyanines, such as indocyanine green, and combinations thereof. These, as well as other photoactive compounds, are described in U.S. Patent Nos. 5,028,621; 4,866,168; 4,935,498; 4,649,151; 5,438,071; 5,198,460; 5,002,962; 5,093,349; 5,171,741; 5,173,504; 4,968,715; 5,190,966; 5,314,905; 5,587,371; 5,798,349; 5,587,479; 6,225,303; U.S. Publication No. 2002.0094998, and PCT Publication No. WO 01/58240, the entire disclosure of each of which being incorporated herein by reference.

Preferably, photoactive agents useful in the methods of the invention comprise compounds that may be administered to a patient without causing any substantial undesirable side effects, and that absorb wavelengths of electromagnetic radiation transmitted from a suitable source, such as laser, that do not cause undesirable thermal damage. In other words, the effects provided by the laser treatment are due primarily to the generation of reactive molecules from the photoactive compound by absorption of energy from the laser.

The step of introducing a photoactive agent may comprise any suitable means for introducing the photoactive agent into the eye. For example, the step of introducing may include administering to a patient, an amount of a photoactive agent to permit an effective amount of the photoactive agent such that the agent will localize in the eye, particularly the retinal cells of the eye. For example, the photoactive agent may be introduced systemically, for example intravenously. The photoactive agent may be introduced intravenously either as a bolus, as a slow infusion, or as a fast infusion.

The step of irradiating the eye in order to activate the photoactive agent preferably comprises exposing or subjecting the eye to electromagnetic radiation, for example light energy, effective in activating the agent. The electromagnetic radiation may comprise radiation have a desired wavelength selected for activating the photoactive agent in the eye, depending upon the type of photoactive agent used.

Preferably, the administration of photodynamic therapy is accomplished during a period of time in which the implant is located, for example, fixed in the eye in order to obtain the most effective, most beneficial treatment. Thus, the present methods may include introducing the photodynamic agent into the eye subsequent to, for example within about one hour, within about six hours, within about one day, or within about one week or more of the implantation of the implant into the eye. For example, the step of irradiating may occur at a time in which both the photodynamic agent and the implant are located in the eye.

Preferably, the uses provide for extended release times of the anti-inflammatory component from the implant placed in the eye. Thus, the patient in whose eye the implant has been placed receives a therapeutic amount of an anti-inflammatory agent for a long or extended time period without requiring additional administrations of the agent. For example, the patient has a substantially consistent level of anti-inflammatory agent available for consistent treatment of the eye over an extended or sustained period of time, for example, on the order of at least about one month, such as between about two and about six months, or even for about one or about two years or longer after receiving an implant. Such extended release times facilitate obtaining successful treatment results.

Our invention also includes is the improvment of vision by: placing into the vitreous of an eye of a patient with macular degeneration a biodegradable implant comprising a poly lactic acid poly glycolic acid copolymer (PLGA) and an anti-inflammatory active agent associated with the PLGA, followed by ; introducing a photoactive agent into the eye, and then; irradiating the eye to activate the photoactive agent, thereby treating the macular degeneration and improving the patient's vision. The photoactive agent can be intravenously administered. The photoactive agent can be a porphyrin, verteporfin, hematoporphyrins, hematoporphyrin derivatives, pheophorbides, derivatives of pheophorbides, bacteriochlorins, purpurins, merocyanines, porphycenes, and combinations thereof.

The active agent used for improving vision can be a steroid. Additionally, the anti-inflammatory active agent can be a cortisone, dexamethasone, fluocinolone, hydrocortisone, methylprednisolone, prednisolone, prednisone, or triamcinolone, or derivatives thereof and mixtures thereof. For example, the anti-inflammatory active agent can be a corticosteroid, such as dexamethasone.

A detailed embodiment of the present invention is the improvement of vision by: (a) placing into the vitreous of an eye of a patient with macular degeneration a biodegradable implant comprising a polylactic acid, polyglycolic acid copolymer (PLGA) and an anti-inflammatory steroid associated with the PLGA; (b) intravenously introducing a porphyrin photoactive agent into the eye, and; (c) irradiating the eye to activate the photoactive agent, thereby treating the macular degeneration and improving the patient's vision.

A alternate detailed embodiment of the present invention is the treatment of subfoveal choroidal neovascularization by: (a) placing into the vitreous of an eye of a patient with subfoveal choroidal neovascularization a biodegradable implant comprising a polylactic acid, polyglycolic acid copolymer (PLGA) and dexamethasone associated with the PLGA; (b) intravenously introducing a porphyrin photoactive agent into the eye, and; (c) irradiating the eye to activate the photoactive agent, thereby treating the subfoveal choroidal neovascularization by reducing the incidence of the subfoveal choroidal neovascularization in the eye of the patient by an amount greater than the reduction of an incidence of subfoveal choroidal neovascularization in a reference (i.e. a control eye upon which a method comprising only step (a) or only steps (b) and (c) has been practised.

Each and every feature described herein, and each and every combination of two or more of such features, is included within the scope of the present invention provided that the features included in such a combination are not mutually inconsistent. In addition, any feature or combination of features may be specifically excluded from any embodiment of the present invention.

Additional aspects and advantages of the present invention are set forth in the following description and claims.

### DESCRIPTION

Generally, the present invention provides methods for treating an eye using photodynamic therapy in conjunction with a beneficial drug delivery system.

The present invention is especially effective for treating conditions of the eye characterized, at least in part, by retinal abnormalities, for example, characterized by choroidal neovascularization (CNV). Such conditions include, for example, neovascularization in age-related macular degeneration and macular edema, ocular histoplasmosis syndrome, pathologic myopia, angioid streaks, idiopathic disorders, choroiditis, choroidal rupture, overlying choroids nevi, and certain inflammatory diseases and disorders.

### Definitions

For the purposes of this description, we use the following terms as defined in this section, unless the context of the word indicates a different meaning.

As used herein, an "intraocular implant" refers to a device or element that is structured, sized, or otherwise configured to be placed in an eye. Intraocular implants are generally biocompatible with physiological conditions of an eye and do not cause adverse side effects. Intraocular implants may be placed in an eye without disrupting vision of the eye.

As used herein, a "therapeutic component" refers to a portion of an intraocular implant comprising one or more therapeutic agents or substances used to treat a medical condition of the eye. The therapeutic component may be a discrete region of an intraocular implant, or it may be homogenously distributed throughout the implant. The therapeutic agents of the therapeutic component are typically ophthalmically acceptable, and are provided in a form that does not cause adverse reactions when the implant is placed in an eye.

As used herein, a "drug release sustaining component" refers to a portion of the intraocular implant that is effective to provide a sustained release of the therapeutic agents of the implant. A drug release sustaining component may be a biodegradable polymer matrix, or it may be a coating covering a core region of the implant that comprises a therapeutic component.

As used herein, "associated with" means mixed with, dispersed within, coupled to, covering, or surrounding. With respect to intraocular implants which comprise a therapeutic component associated with a biodegradable polymer matrix, "associated with" specifically excludes biodegradable polymeric coatings that may be provided on or around the matrix.

As used herein, an "ocular region" or "ocular site" refers generally to any area of the eyeball, including the anterior and posterior segment of the eye, and which generally includes, but is not limited to, any functional (e.g., for vision) or structural tissues found in the eyeball, or tissues or cellular layers that partly or completely line the interior or exterior of the eyeball. Specific examples of areas of the eyeball in an ocular region include the anterior chamber, the posterior chamber, the vitreous cavity, the choroid, the suprachoroidal space, the conjunctiva, the subconjunctival space, the episcleral space, the intracorneal space, the epicorneal space, the sclera, the pars plana, surgically-induced avascular regions, the macula, and the retina.

As used herein, an "ocular condition" is a disease, ailment or condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball.

An anterior ocular condition is a disease, ailment or condition which affects or which involves an anterior (i.e. front of the eye) ocular region or site, such as a periocular muscle, an eye lid or an eye ball tissue or fluid which is located anterior to the posterior wall of the lens capsule or ciliary muscles. Thus, an anterior ocular condition primarily affects or involves the conjunctiva, the cornea, the anterior chamber, the iris, the posterior chamber (behind the retina but in front of the posterior wall of the lens capsule), the lens or the lens capsule and blood vessels and nerve which vascularize or innervate an anterior ocular region or site.

Thus, an anterior ocular condition can include a disease, ailment or condition, such as for example, aphakia; pseudophakia; astigmatism; blepharospasm; cataract; conjunctival diseases; conjunctivitis; corneal diseases;, corneal ulcer; dry eye syndromes; eyelid diseases; lacrimal apparatus diseases; lacrimal duct obstruction; myopia; presbyopia; pupil disorders; refractive disorders and strabismus. Glaucoma can also be considered to be an anterior ocular condition because a clinical goal of glaucoma treatment can be to reduce a hypertension of aqueous fluid in the anterior chamber of the eye (i.e. reduce intraocular pressure).

A posterior ocular condition is a disease, ailment or condition which primarily affects or involves a posterior ocular region or site such as choroid or sclera (in a position posterior to a plane through the posterior wall of the lens capsule), vitreous, vitreous chamber, retina, optic nerve (i.e. the optic disc), and blood vessels and nerves which vascularize or innervate a posterior ocular region or site.

Thus, a posterior ocular condition can include a disease, ailment or condition, such as for example, acute macular neuroretinopathy; Behcet's disease; choroidal neovascularization; diabetic uveitis; histoplasmosis; infections, such as fungal or viral-caused infections; macular degeneration, such as acute macular degeneration, non-exudative age related macular degeneration and exudative age related macular degeneration; edema, such as macular edema, cystoid macular edema and diabetic macular edema; multifocal choroiditis; ocular trauma which affects a posterior ocular site or location; ocular tumors; retinal disorders, such as central retinal vein occlusion, diabetic retinopathy (including proliferative diabetic retinopathy), proliferative vitreoretinopathy (PVR), retinal arterial occlusive disease, retinal detachment, uveitic retinal disease; sympathetic opthalmia; Vogt Koyanagi-Harada (VKH) syndrome; uveal diffusion; a posterior ocular condition caused by or influenced by an ocular laser treatment; posterior ocular conditions caused by or influenced by a photodynamic therapy, photocoagulation, radiation retinopathy, epiretinal membrane disorders, branch retinal vein occlusion, anterior ischemic optic neuropathy, non-retinopathy diabetic retinal dysfunction, retinitis pigmentosa, and glaucoma. Glaucoma can be considered a posterior ocular condition because the therapeutic goal is to prevent the loss of or reduce the occurrence of loss of vision due to damage to or loss of retinal cells or optic nerve cells (i.e. neuroprotection).

The term "biodegradable polymer" refers to a polymer or polymers which degrade in vivo, and wherein erosion of the polymer or polymers over time occurs concurrently with or subsequent to release of the therapeutic agent. Specifically, hydrogels such as methylcellulose which act to release drug through polymer swelling are specifically excluded from the term "biodegradable polymer". The terms "biodegradable" and "bioerodible" are equivalent and are used interchangeably herein. A biodegradable polymer may be a homopolymer, a copolymer, or a polymer comprising more than two different polymeric units.

The term "treat", "treating", or "treatment" as used herein, refers to reduction or resolution or prevention of an ocular condition, ocular injury or damage, or to promote healing of injured or damaged ocular tissue.

The term "therapeutically effective amount" as used herein, refers to the level or amount of agent needed to treat an ocular condition, or reduce or prevent ocular injury or damage without causing significant negative or adverse side effects to the eye or a region of the eye.

As described herein, the methods of the present invention generally comprise the steps of placing a drug delivery system element for example, and intraocular implant into an eye, and subjecting the eye to photodynamic therapy.

The drug delivery system element (hereinafter "implant") is preferably an extended release drug delivery implant that provides one or more benefits to an eye in which it is placed. The implant may be at least partially bioerodible and comprises an anti-inflammatory component and a bioerodible polymeric component. Other implants used in conjunction with photodynamic therapy may have a non-biodegradable polymeric outer coating with one or more openings structured to permit a therapeutic agent to pass therethrough, such as the implants disclosed in U.S. Patent No. 6,331,313.

The anti-inflammatory component of the implant comprises one or more anti-inflammatory agents, such as steroidal anti-inflammatory agents or non-steroidal anti-inflammatory agents. In some embodiments of the invention, the anti-inflammatory component comprises a therapeutically active agent selected from the group consisting of cortisone, dexamethasone, fluocinolone, hydrocortisone, methylprednisolone, prednisolone, prednisone, and triamcinolone, derivatives thereof and mixtures thereof. The anti-inflammatory agent may be selected from the group consisting of corticosteroids and mixtures thereof. Preferably, the anti-inflammatory component is dexamethasone.

In some embodiments of the invention, the bioerodible implant comprises an ophthalmically acceptable therapeutic agent in addition to the anti-inflammatory component. For example, the implant may include, in addition to the anti-inflammatory agent, an antiviral agent, an antibiotic agent, an antifungal agent, an anti-cancer agent, an antiglaucoma agent, an analgesic, an immunomodulatory agent, a macro-molecule, or a mixture thereof.

Preferably, the implant is structured such that the anti-inflammatory agent is associated with the bioerodible polymeric component, for example is dispersed within the bioerodible polymeric component, mixed with the bioerodible polymeric component, coupled to the bioerodible polymeric component, covered by the bioerodible polymeric component, or surrounded by the bioerodible polymeric component. For example, an implant used in a method of the invention can be formulated with particles of an active agent dispersed within a biodegradable polymer matrix. Release of the active agent can be achieved by erosion of the biodegradable polymer matrix and by diffusion of the particulate agent into an ocular fluid, for example, vitreal fluid, with contemporaneous or subsequent dissolution of the polymer matrix. Release of the active agent may be controlled based in part on a level of access of ocular fluid to the particulate agent through openings or pores of the element.

The implants may be structured such that the bioerodible polymer is in the form of a matrix material comprising at least about 10 percent, at least about 20 percent, at least about 30 percent, at least about 40 percent, at least about 50 percent, at least about 60 percent, at least about 70 percent, at least about 80 percent, or at least about 90 percent by weight of the implant.

The methods may provide for extended release times of one or more therapeutic agents including the anti-inflammatory agent, from the implant placed in the eye. Thus, the patient in whose eye the implant has been placed receives a therapeutic amount of an agent for a long or extended time period without requiring additional administrations of the agent. For example, the patient has a substantially consistent level of therapeutically active agent available for consistent treatment of the eye over a relatively long period of time, for example, on the order of at least about one month, such as between about two and about six months, or even for about one or about two years or longer after receiving an implant. Such extended release times facilitate obtaining successful treatment results.

The release kinetics of the implants that are useful in the methods of the present invention can be dependent in part on other factors, such as, for example, the surface area of the implant. A larger surface area exposes more of the implant composition to ocular fluid, causing faster erosion of the polymer matrix and faster dissolution of the active agent particles in the fluid. Therefore, the size and shape of the implant may also be used to control the rate of release, period of treatment, and active agent concentration at the site of implantation. At equal active agent loads, larger implants will deliver a proportionately larger dose, but depending on the surface to mass ratio, may possess a slower release rate.

Other factors which influence the release kinetics of active agent from the implant can include such characteristics as the size and shape of the implant, the size of the active agent particles, the solubility of the active agent, the ratio of active agent to polymer(s), the method of manufacture, the surface area exposed, and the erosion rate of the polymer(s). The release kinetics achieved by degradation or erosion of the element are different than that achieved through formulations which release active agents through polymer swelling, such as with crosslinked hydrogels. In that case, the active agent is not released through polymer erosion, but through polymer swelling and drug diffusion, which releases agent as liquid diffuses through the pathways exposed. It is also contemplated that the presence an/or activation of the photoactive agent that has been introduced into the eye in accordance with the present invention may influence the release kinetics of active agent from the implant.

It is additionally noted that the release rate of the active agent from implants used in the methods in accordance with the invention can in some embodiments depend at least in part on the mechanism of degradation of the polymeric component or components making up the biodegradable polymer matrix. For example, condensation polymers may be degraded by hydrolysis (among other mechanisms) and therefore any change in the composition of the implant that enhances water uptake by the implant will likely increase the rate of hydrolysis, thereby increasing the rate of polymer degradation and erosion, and thus increasing the rate of active agent release.

The implants used in the methods in accordance with the invention may be of any geometry including particles, sheets, patches, plaques, films, discs, fibers, rods, and the like, or may be of any size or shape compatible with the selected site of implantation, as long as the implants have the desired release kinetics and deliver an amount of anti-inflammatory agent and in some instances, one or more other active agents that will be therapeutic for the intended medical condition of the eye. An upper limit for the implant size will be determined by factors such as the desired release kinetics, toleration for the implant at the site of implantation, size limitations on insertion, and ease of handling. For example, the vitreous chamber is able to accommodate relatively large rod-shaped implants, generally having diameters of about 0.05 mm to 3 mm and a length of about 0.5 to about 10 mm. In one variation, the rods have diameters of about 0.1 mm to about 1 mm. In another variation, the rods have diameters of about 0.3 mm to about 0.75 mm. In yet a further variation, other implants having variable geometries but approximately similar volumes may also be used.

Preferably, the bioerodible polymeric component of the implant useful in the methods of the present invention, comprises one or more types of bioerodible polymers. For example, the bioerodible polymeric component may comprise a mixture of a first biodegradable polymer having terminal acid groups, and a second biodegradable polymer having terminal acid groups. For example, the first biodegradable polymer may comprise a poly (D,L-lactide-co-glycolide).and the second biodegradable polymer may comprise a poly (D,L-lactide).

In some embodiments of the invention, the bioerodible polymeric component of the implant used in the methods for treating an eye includes a polymeric material selected from the group consisting of a polymer of poly-lactic acid, a polymer of poly-glycolic acid, a copolymer of lactic acid and glycolic acid, and combinations thereof.

In certain embodiments, the implant comprises an anti-inflammatory steroid, preferably dexamethasone, dispersed within a PLGA polymeric matrix material.

Preferably, the step of subjecting the eye to photodynamic therapy is performed concurrently with release of the anti-inflammatory agent into the eye. The photodynamic therapy may be accomplished in any suitable manner known in the art for treating the eye, for example for treating CNV. More particularly, the step of subjecting the eye to photodynamic therapy may comprise introducing a photoactive agent, for example, a photoactive compound, into the eye, and irradiating the eye to activate the photoactive agent.

The photoactive agent is preferably a chemical compound that, when introduced into the bloodstream of the patient, accumulates within retinal cells of an eye, and when exposed to electromagnetic energy, for example laser irradiation, becomes activated thereby. The photoactive agent may be used both diagnostically, such as for identifying areas of neovascularization, and therapeutically, such as for causing coagulation or other tissue reaction when exposed to light energy. For example in some embodiments of the invention, the photoactive agent may be effective to form, one or more reactive ion species, such as free radicals, when the photoactive agent is exposed to particular wavebands or particular wavelengths of light. These reactive ion species are effective in destruction of unwanted neovascularization in the retina.

Examples of suitable photoactive agents for purposes of the present invention include, but are not limited to, porphyrins, hematoporphyrins, hematoporphyrin derivatives, pheophorbides, derivatives of pheophorbides, benzoporphyrins, benzoporphyrin derivatives, such as verteporfin, bacteriochlorins, purpurins, merocyanines, porphycenes, tricarbocyanines, such as indocyanine green, and combinations thereof. In certain implants, the photoactive agent comprises porphyrin or verteporfin. These, as well as other photoactive compounds, are described in U.S. Patent Nos. 5,028,621; 4,866,168; 4,935,498; 4,649,151; 5,438,071; 5,198,460; 5,002,962; 5,093,349; 5,171,741; 5,173,504; 4,968,715; 5,190,966; 5,314,905; 5,587,371; 5,798,349; 5,587,479; 6,225,303; U.S. Publication No. 2002.0094998, and PCT Publication No. WO 01/58240, the entire disclosure of each of which being incorporated herein by reference.

Preferably photoactive agents useful in the methods of the invention comprise agents that may be administered to a patient without causing any substantial undesirable side effects, and that absorb wavelengths of electromagnetic radiation transmitted from a suitable source, such as laser, that do not cause undesirable thermal damage. In other words, the effects provided by the laser treatment are due primarily to the generation of reactive molecules from the photoactive agent by absorption of energy from the laser.

The dosage of the photoactive agent that is administered to a patient may vary, according to the activity of the specific agent chosen, the formulation, and whether the agent is joined to a carrier and thus targeted to a specific tissue. When using green porphyrins, dosages are usually in the range of 0.1-50 mg/M² of body surface area; more preferably from about 1-10 mg/M² or from about 2-8 mg/M². Parameters to be considered when determining the dosage include the duration and wavelength of the light irradiation, the nature of the photochemical reaction induced by the light irradiation, and the dye-to-laser time period.

The step of introducing a photoactive agent into the eye may include administering to a patient an amount of a photoactive agent effective to permit the photoactive agent to localize in the eye, particularly in or near the retinal cells of the eye. For example, the photoactive agent may be introduced systemically, for example intravenously. For example, the photoactive agent may be introduced intravenously either as a bolus, as a slow infusion over an extended period of time, or a relatively faster infusion over a relatively shorter period of time.

In accordance with the invention, electromagnetic radiation is directed to a target site in the eye for a sufficient time after the administration of the photodynamic agent so as to permit the photodynamic agent to reach its target tissue. Upon being irradiated with the wavelength(s) appropriate to the photodynamic agent or agents chosen, the agent enters an excited state and is thought to interact with other compounds in the tissue to form highly reactive intermediates which can then destroy the target endothelial tissue, causing platelet aggregation and thrombosis. Fluence of the irradiation may vary depending on factors such as the depth of tissue to be treated and the tissue type--generally it is between about 25 and about 200 Joules/cm². Irradiance typically is between about 150 and about 900 mW/cm², but can also vary somewhat from this range.

Light treatment may be given as soon as about 5 minutes following administration of the photodynamic agent and up to about 2 hours to about 6 hours or more after administration of the agent.

The step of introducing a photoactive agent may comprise any suitable means for introducing the photoactive agent into the eye. For example, the step of introducing may include administering to a patient, an amount of a photoactive agent effective to cause the photoactive agent to reach an effective concentration of the agent within the eye, particularly within the capillaries of the retinal cells of the eye. For example, the photoactive agent may be introduced systemically, for example intravenously. The photoactive agent may be introduced intravenously either as a bolus, as a slow infusion, or as a fast infusion.

The step of irradiating the eye in order to activate the photoactive agent preferably comprises exposing or subjecting the eye to electromagnetic radiation, for example light energy, effective in activating the agent. The electromagnetic radiation may comprise radiation have a desired wavelength selected for activating the photoactive agent in the eye, depending upon the type of photoactive agent used.

Preferably, the administration of photodynamic therapy is accomplished during a period of time of which the implant is located, for example, implanted, in the eye in order to obtain the most effective, most beneficial treatment. Thus, the present methods may include introducing the photodynamic agent into the eye subsequent to the step of placing the implant in the eye such that the implant is located in the eye during the step of irradiating the eye.

In addition, one or more neuroprotectants may be administered to the patient in conjunction with the photodynamic therapy and the administration of the drug delivery system implant. Neuroprotectants may be administered separately, or may be released from the implant containing the anti-inflammatory agent. Neuroprotective agents preferably preserve the cellular, biochemical, and physiological properties of the neurons. Examples of neuroprotective agents include anti-excitotoxic agents, such as glutamate receptor (e.g., NMDA receptor) modulators (such as, MK-801, N4K-801, memantine), calcium channel blockers, and inhibitory receptor modulators (such as GABA receptor agonists, including, but not limited to, anesthetics, such as barbiturates; benzodiazepines, such as zolpidem; and alcohol, such as ethanol). Anti-excitotoxic agents preferably reduce or prevent excessive increases in intracellular calcium concentration. Neuroprotective agents also include adenosine receptor modulators, adrenergic receptor modulators (such as, α2-receptor agonists, brimonidine, beta-blockers, etc.), glutamate uptake modulators, dopamine receptor modulators, ion channel modulators (such as, sodium or hydrogen), downstream intracellular signal modulators (such as, COP-1), prostaglandins (such as EP2 agonists), ryanodine receptor agonists (calcium release from intracellular stores), cytokines, neurotrophic and/or nerve growth factors, such as nerve growth factor (NGF) including NGFα, brain derived neurotrophic factor (BDNF), ciliary neurotrophic factor (CNTF), bone-derived growth factor (BDGF), neurotrophin-3 (NT-3), neurotrophin-4/5 (NT-4/5), pigment epithelium derived factor, vitamin C, cyclosporins, drugs that are active in ischemia/reperfusion assays, monoamine oxidase inhibitors (MAOIs), carbonic anhydrase inhibitors (such as acetazolamide), pump inhibitors (such as, amiloride), free-radical scavengers, nitric oxide synthetase inhibitors, and hormones.

Intraocular implants suitable for use in the methods of the invention preferably comprise a therapeutic component associated with a biodegradable polymeric material. In a preferred embodiment of the invention, the therapeutic component comprises an anti-inflammatory agent, for example, but not limited to a steroid. Preferably the implant is structured such that the therapeutically effective amount of the anti-inflammatory agent is released into the eye for a period of time greater than about one week, or about one month, or about six months after the implant is placed in the eye.

The implants are effective to provide a therapeutically effective dosage of the therapeutic agent or agents directly to a region of the eye to treat one or more undesirable ocular conditions. Thus, with a single administration, therapeutic agents will be made available at the site where they are needed and will be maintained for an extended period of time, rather than subjecting the patient to repeated injections or, in the case of self-administered drops, ineffective treatment with only limited bursts of exposure to the active agent or agents.

In one embodiment of the present invention, an intraocular implant comprises a biodegradable polymer matrix. The biodegradable polymer matrix degrades at a rate effective to sustain release of a therapeutically effective amount of the anti-inflammatory agent for a time greater than about one week, or about one month, or about three months from the time in which the implant is placed in ocular region or ocular site, such as the vitreous of an eye.

The anti-inflammatory component may comprise a corticosteroid. In certain embodiments, the anti-inflammatory component comprises dexamethasone, fluocinolone, triamcinolone, or a mixture thereof. In some embodiments, the fluocinolone is provided in the implant as fluocinolone acetonide, and the triamcinolone is provided in the implant as triamcinolone acetonide. Triamcinolone acetonide is publicly available under the tradename, KENALOG®.

The anti-inflammatory component may be in a particulate or powder form and entrapped by the biodegradable polymer matrix. Usually, steroid particles will have an effective average size less than about 3000 nanometers. In certain implants, the particles may have an effective average particle size about an order of magnitude smaller than 3000 nanometers. For example, the particles may have an effective average particle size of less than about 500 nanometers. In additional implants, the particles may have an effective average particle size of less than about 400 nanometers, and in still further embodiments, a size less than about 200 nanometers.

The anti-inflammatory component of the implant is preferably from about 10 to 90% by weight of the implant. More preferably, the anti-inflammatory component is from about 50 to about 80% by weight of the implant. In a preferred embodiment, the anti-inflammatory component comprises about 50% by weight of the implant. In another embodiment, the anti-inflammatory component comprises about 70% by weight of the implant.

Suitable polymeric materials or compositions for use in the implant include those materials which are compatible, that is biocompatible, with the eye so as to cause no substantial interference with the functioning or physiology of the eye. Such materials preferably are at least partially and more preferably substantially completely biodegradable or bioerodible.

Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than about 5%, or less than about 1% of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, "Biodegradable Polymers in Controlled Drug Delivery", in: CRC Critical Reviews in Therapeutic Drug Carrier Systems, Vol. 1, (CRC Press, Boca Raton, FL 1987), pp 39-90, which describes encapsulation for controlled drug delivery, may find use in the present implants.

Of additional interest are polymers of hydroxyaliphatic carboxylic acids, either homopolymers or copolymers, and polysaccharides. Polyesters of interest include polymers of D-lactic acid, L-lactic acid, racemic lactic acid, glycolic acid, polycaprolactone, and combinations thereof. Generally, by employing the L-lactate or D-lactate, a slowly eroding polymer or polymeric material is achieved, while erosion is substantially enhanced with the lactate racemate.

Among the useful polysaccharides are, without limitation, calcium alginate, and functionalized celluloses, particularly carboxymethylcellulose esters characterized by being water insoluble, a molecular weight of about 5 kD to 500 kD, for example.

Other polymers of interest include, without limitation, polyvinyl alcohol, polyesters, polyethers and combinations thereof which are biocompatible and may be biodegradable and/or bioerodible.

Some preferred characteristics of the polymers or polymeric materials for use in the present invention may include biocompatibility, compatibility with the therapeutic component, ease of use of the polymer in making the drug delivery systems of the present invention, a half-life in the physiological environment of at least about 6 hours, preferably greater than about one day, not significantly increasing the viscosity of the vitreous, and water insolubility.

The biodegradable polymeric materials useful for forming a matrix of the implant are desirably subject to enzymatic or hydrolytic instability. Water soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer and hence the extended release profile of the implant is the relative average molecular weight of the polymeric composition employed in the implant. Different molecular weights of the same or different polymeric compositions may be included in the implant to modulate the release profile. In certain implants, the relative average molecular weight of the polymer will range from about 9 to about 60 kD, usually from about 10 to about 54 kD, more usually from about 12 to about 45 kD, and most usually less than about 40 kD.

In some implants useful in the methods of the invention, copolymers of glycolic acid and lactic acid are used, where the rate of biodegradation is controlled by the ratio of glycolic acid to lactic acid. The most rapidly degraded copolymer has roughly equal amounts of glycolic acid and lactic acid. Homopolymers, or copolymers having ratios other than equal, are more resistant to degradation. The ratio of glycolic acid to lactic acid will also affect the brittleness of the implant, where a more flexible implant is desirable for larger geometries. The % of polylactic acid in the polylactic acid polyglycolic acid (PLGA) copolymer can be 0-100%, preferably about 15-85%, more preferably about 35-65%. In some implants, a 50/50 PLGA copolymer is used.

The biodegradable polymer matrix of the intraocular implant may comprise a mixture of two or more biodegradable polymers. For example, the implant may comprise a mixture of a first biodegradable polymer and a different second biodegradable polymer. One or more of the biodegradable polymers may have terminal acid groups. In certain implants, the matrix comprises a first biodegradable polymer having terminal acid groups, and a different second biodegradable polymer having terminal acid groups. The first biodegradable polymer may be a poly (D,L-lactide-co-glycolide). The second biodegradable polymer may be a poly (D,L-lactide).

Release of a therapeutic agent, such as an anti-inflammatory agent, from an erodible polymer is the consequence of several mechanisms or combinations of mechanisms. Some of these mechanisms include desorption from the implants surface, dissolution, diffusion through porous channels of the hydrated polymer and erosion. Erosion can be bulk or surface or a combination of both. As discussed herein, the matrix of the intraocular implant may release drug at a rate effective to sustain release of a therapeutically effective amount of the steroid for more than three months after implantation into an eye. In certain implants, therapeutic amounts of the steroid are released for more than four months after implantation. For example, an implant may comprise fluocinolone, and the matrix of the implant degrades at a rate effective to sustain release of a therapeutically effective amount of fluocinolone for about three months after being placed in an eye. As another example, the implant may comprise triamcinolone, and the matrix releases drug at a rate effective to sustain release of a therapeutically effective amount of triamcinolone for more than three months, such as from about three months to about six months.

One preferred example of the biodegradable intraocular implant useful in accordance with methods of the invention comprises the anti-inflammatory dexamethasone associated with a biodegradable polymer matrix, which comprises a mixture of different biodegradable polymers. At least one of the biodegradable polymers is a polylactide having a molecular weight less than 40 kD. Such a mixture is effective in sustaining release of a therapeutically effective amount of the steroid for a time period greater than about two months from the time the implant is placed in an eye. In certain embodiments, the polylactide has a molecular weight less than 20 kD. In other embodiments, the polylactide has a molecular weight of about 10 kD. The polylactide may be a poly (D,L-lactide), and the polylactide may include polymers having terminal free acid groups. In one particular embodiment, the matrix of the implant comprises a mixture of poly(lactide-co-glycolide) and polylactide. Each of the poly(lactide-co-glycolide) and polylactide may have terminal free acid groups.

Another example of a biodegradable intraocular implant comprises an anti-inflammatory agent such as dexamethasone associated with a biodegradable polymer matrix, which comprises a mixture of different biodegradable polymers, each biodegradable polymer having an inherent viscosity from about 0.16 dl/g to about 0.24 dl/g. For example, one of the biodegradable polymers may have an inherent viscosity of about 0.2 dl/g. Or, the mixture may comprise two different biodegradable polymers, and each of the biodegradable polymers has an inherent viscosity of about 0.2 dl/g. The inherent viscosities identified above may be determined in 0.1 % chloroform at 25°C.

Other implants useful in the methods of the present invention may include a biodegradable polymer matrix of biodegradable polymers, at least one of the polymers having an inherent viscosity of about 0.25 dl/g to about 0.35 dl/g. Additional implants may comprise a mixture of biodegradable polymers wherein each polymer has an inherent viscosity from about 0.50 dl/g to about 0.70 dl/g.

The release of the anti-inflammatory agent from the intraocular implant comprising a biodegradable polymer matrix may include an initial burst of release followed by a gradual increase in the amount of the anti-inflammatory component released, or the release may include an initial delay in release of the anti-inflammatory component followed by an increase in release. When the implant is substantially completely degraded, the percent of the anti-inflammatory component that has been released is about one hundred. Compared to existing implants, the implants disclosed herein do not completely release, or release about 100% of the steroid, until after about two months of being placed in an eye. Thus, the implants exhibit a cumulative release profile that may have a shallower slope, or a lower rate of release, for longer periods of time than existing implants.

It may be desirable to provide a relatively constant rate of release of the anti-inflammatory agent from the implant over the life of the implant. For example, it may be desirable for the anti-inflammatory component to be released in amounts from about 0.01 µg to about 2 µg per day for the life of the implant. However, the release rate may change to either increase or decrease depending on the formulation of the biodegradable polymer matrix. In addition, the release profile of the steroid may include one or more linear portions and/or one or more non-linear portions. Preferably, the release rate is greater than zero once the implant has begun to degrade or erode.

The implants may be monolithic, i.e. having the active agent or agents homogenously distributed through the polymeric matrix, or encapsulated, where a reservoir of active agent is encapsulated by the polymeric matrix. Due to ease of manufacture, monolithic implants are usually preferred over encapsulated forms. However, the greater control afforded by the encapsulated, reservoir-type implant may be of benefit in some circumstances, where the therapeutic level of the drug falls within a narrow window. In addition, the therapeutic component, including the steroid, may be distributed in a non-homogenous pattern in the matrix. For example, the implant may include a portion that has a greater concentration of the anti-inflammatory component relative to a second portion of the implant.

In another embodiment of the present invention, an intraocular implant comprises a therapeutic component, including an anti-inflammatory component, and a drug release sustaining component including a coating covering a core region of the implant. The therapeutic anti-inflammatory component is provided in the core region. The polymeric outer layer may be impermeable to the therapeutic component and ocular fluids. Or, the polymeric outer layer may be initially impermeable to the therapeutic component and ocular fluids, but then may become permeable to the therapeutic component or ocular fluids as the outer layer degrades. Thus, the polymeric outer layer may comprise a polymer such as polytetrafluoroethylene, polyfluorinated ethylenepropylene, polylactic acid, polyglycolic acid, silicone, or mixtures thereof.

The foregoing implant may be understood to include a reservoir of one or more therapeutic agents, such as an anti-inflammatory agent. One example of an implant including a reservoir of a therapeutic agent is described in U.S. Patent No. 6,331,313.

In some implants, the drug release sustaining component comprises a polymeric outer layer covering the therapeutic component, the outer layer comprises a plurality of openings or holes through which the therapeutic component may pass from the drug delivery system to an external environment of the implant, such as an ocular region of an eye. The holes enable a liquid to enter into the interior of the implant and dissolve the therapeutic agent contained therein. The release of the therapeutic agent from the implant may be influenced by the drug solubility in the liquid, the size of the hole(s), and the number of holes. In certain implants, the hole size and number of holes are effective in providing substantially all of the desired release characteristics of the implant. Thus, additional excipients may not be necessary to achieve the desired results. However, in other implants, excipients may be provided to further augment the release characteristics of the implant.

Various biocompatible substantially impermeable polymeric compositions may be employed in preparing the outer layer of the implant. Some relevant factors to be considered in choosing a polymeric composition include: compatibility of the polymer with the biological environment of the implant, compatibility of the drug with the polymer, ease of manufacture, a half-life in the physiological environment of at least several days, no significant enhancement of the viscosity of the vitreous, and the desired rate of release of the drug. Depending on the relative importance of these characteristics, the compositions can be varied. Several such polymers and their methods of preparation are well-known in the art. See, for example, U.S. Pat. Nos. 4,304,765; 4,668,506 4,959,217; 4,144,317, and 5,824,074, Encyclopedia of Polymer Science and Technology, Vol. 3, published by Interscience Publishers, Inc., New York, latest edition, and Handbook of Common Polymers by Scott, J. R. and Roff, W. J., published by CRC Press, Cleveland, Ohio, latest edition.

The polymers of interest may be homopolymers, copolymers, straight, branched-chain, or cross-linked derivatives. Some exemplary polymers include: polycarbamates or polyureas, cross-linked poly(vinyl acetate) and the like, ethylene-vinyl ester copolymers having an ester content of 4 to 80% such as ethylene-vinyl acetate (EVA) copolymer, ethylene-vinyl hexanoate copolymer, ethylene-vinyl propionate copolymer, ethylene-vinyl butyrate copolymer, ethylene-vinyl pentantoate copolymer, ethylene-vinyl trimethyl acetate copolymer, ethylene-vinyl diethyl acetate copolymer, ethylene-vinyl 3-methyl butanoate copolymer, ethylene-vinyl 3-3-dimethyl butanoate copolymer, and ethylene-vinyl benzoate copolymer, or mixtures thereof.

Additional examples include polymers such as: poly(methylmethacrylate), poly(butylmethacrylate), plasticized poly(vinylchloride), plasticized poly(amides), plasticized nylon, plasticized soft nylon, plasticized poly(ethylene terephthalate), natural rubber, silicone, poly(isoprene), poly(isobutylene), poly(butadiene), poly(ethylene), poly(tetrafluoroethylene), poly(vinylidene chloride), poly(acrylonitrile, cross-linked poly(vinylpyrrolidone), chlorinated poly(ethylene), poly(trifluorochloroethylene), poly(ethylene chlorotrifluoroethylene), poly(tetrafluoroethylene), poly(ethylene tetrafluoroethylene), poly(4,4'-isopropylidene diphenylene carbonate), polyurethane, poly(perfluoroalkoxy), poly(vinylidenefluoride), vinylidene chloride-acrylonitrile copolymer, vinyl chloride-diethyl fumarate copolymer, silicone, silicone rubbers (of medical grade such as Silastic® Medical Grade ETR Elastomer Q7-4750 or Dow Corning® MDX 4-4210 Medical Grade Elastomer); and cross-linked copolymers of polydimethylsilane silicone polymers.

Some further examples of polymers include: poly(dimethylsiloxanes), ethylene-propylene rubber, silicone-carbonate copolymers, vinylidene chloride-vinyl chloride copolymer, vinyl chloride-acrylonitrile copolymer, vinylidene chloride-acrylonitrile copolymer, poly(olefins), poly(vinyl-olefins), poly(styrene), poly(halo-olefins), poly(vinyls) such as polyvinyl acetate, cross-linked polyvinyl alcohol, cross-linked polyvinyl butyrate, ethylene ethylacrylate copolymer, polyethyl hexylacrylate, polyvinyl chloride, polyvinyl acetates, plasiticized ethylene vinylacetate copolymer, polyvinyl alcohol, polyvinyl acetate, ethylene vinylchloride copolymer, polyvinyl esters, polyvinylbutyrate, polyvinylformal, poly(acrylate), poly(methacrylate), poly(oxides), poly(esters), poly(amides), and poly(carbonates), or mixtures thereof.

In some aspects, the implants with an outer layer coating with orifices or holes may be biodegradable wherein the outer layer degrades after the drug has been released for the desired duration. The biodegradable polymeric compositions may comprise any of the above-identified biodegradable polymers or combinations thereof. In some implants, the polymer is polytetrafluoroethylene, (commercially known as Teflon®), ethyl vinyl alcohol or ethylene vinyl acetate.

Orifices and equipment for forming orifices are disclosed in U.S. Pat. Nos. 3,845,770; 3,916,899; 4,063,064 and 4,008,864. Orifices formed by leaching are disclosed in U.S. Pat. Nos. 4,200,098 and 4,285,987. Laser drilling machines equipped with photo wave length detecting systems for orienting a device are described in U.S. Pat. No. 4,063,064 and in U.S. Pat. No. 4,088,864.

The intraocular implants may have a size of between about 5 µm and about 10 mm, or between about 10 µm and about 1 mm for administration with a needle, greater than 1 mm, or greater than 2 mm, such as 3 mm or up to 10 mm, for administration by surgical implantation. For needle-injected implants, the implants may have any appropriate length so long as the diameter of the implant permits the implant to move through a needle. For example, implants having a length of about 6 mm to about 7 mm have been injected into an eye. The implants administered by way of a needle should have a diameter that is less than the inner diameter of the needle. In certain implants, the diameter is less than about 500 µm. The vitreous chamber in humans is able to accommodate relatively large implants of varying geometries, having lengths of, for example, 1 to 10 mm. The implant may be a cylindrical pellet (e. g., rod) with dimensions of about 2 mm x 0.75 mm diameter. Or the implant may be a cylindrical pellet with a length of about 7 mm to about 10 mm, and a diameter of about 0.75 mm to about 1.5 mm.

The implants may also be at least somewhat flexible so as to facilitate both insertion of the implant in the eye, such as in the vitreous, and accommodation of the implant. The total weight of the implant is usually about 250-5000 µg, more preferably about 500-1000 µg. For example, an implant may be about 500 µg, or about 1000 µg. For non-human individuals, the dimensions and total weight of the implant(s) may be larger or smaller, depending on the type of individual. For example, humans have a vitreous volume of approximately 3.8 ml, compared with approximately 30 ml for horses, and approximately 60-100 ml for elephants. An implant sized for use in a human may be scaled up or down accordingly for other animals, for example, about 8 times larger for an implant for a horse, or about, for example, 26 times larger for an implant for an elephant.

Thus, implants can be prepared where the center may be of one material and the surface may have one or more layers of the same or a different composition, where the layers may be cross-linked, or of a different molecular weight, different density or porosity, or the like. For example, where it is desirable to quickly release an initial bolus of drug, the center may be a polylactate coated with a polylactate-polyglycolate copolymer, so as to enhance the rate of initial degradation. Alternatively, the center may be polyvinyl alcohol coated with polylactate, so that upon degradation of the polylactate exterior the center would dissolve and be rapidly washed out of the eye.

The implants, particularly the implants with the anti-inflammatory component associated with a biodegradable polymer matrix, may be of any geometry including fibers, sheets, films, microspheres, spheres, circular discs, plaques and the like. The upper limit for the implant size will be determined by factors such as toleration for the implant, size limitations on insertion, ease of handling, etc. Where sheets or films are employed, the sheets or films will be in the range of at least about 0.5 mm x 0.5 mm, usually about 3-10 mm x 5-10 mm with a thickness of about 0.1-1.0 mm for ease of handling. Where fibers are employed, the fiber diameter will generally be in the range of about 0.05 to 3 mm and the fiber length will generally be in the range of about 0.5-10 mm. Spheres may be in the range of about 0.5 µm to 4 mm in diameter, with comparable volumes for other shaped particles.

The size and form of the implant can also be used to control the rate of release, period of treatment, and drug concentration at the site of implantation. Larger implants will deliver a proportionately larger dose, but depending on the surface to mass ratio, may have a slower release rate. The particular size and geometry of the implant are chosen to suit the site of implantation.

A treatment of patient in accordance with the present invention may include placing the implant directly into the posterior chamber of the eye. In other embodiments, a treatment of a patient may comprise administering an implant to the patient by at least one of intravitreal injection, subconjuctival injection, sub-tenon injections, retrobulbar injection, and suprachoroidal injection.

In at least one embodiment, a treatment of a posterior ocular condition comprises administering one or more implants containing one or more steroids, as disclosed herein to a patient by at least one of intravitreal injection, subconjuctival injection, sub-tenon injection, retrobulbar injection, and suprachoroidal injection. A syringe apparatus including an appropriately sized needle, for example, a 22 gauge needle, a 27 gauge needle or a 30 gauge needle, can be effectively used to inject the composition with the posterior segment of an eye of a human or animal. Repeat injections are often not necessary due to the extended release of the steroid from the implants.

In another aspect of the invention, kits for treating an ocular condition of the eye are provided, comprising: a) a container comprising an extended release implant comprising a therapeutic component including a steroid, such as fluocinolone or triamcinolone, and drug release sustaining component; and b) instructions for use. Instructions may include steps of how to handle the implants, how to insert the implants into an ocular region, and what to expect from using the implants.

The proportions of anti-inflammatory, polymer, and any other modifiers may be empirically determined by formulating several implants with varying proportions. A USP approved method for dissolution or release test can be used to measure the rate of release (USP 23; NF 18 (1995) pp. 1790-1798). For example, using the infinite sink method, a weighed sample of the implant is added to a measured volume of a solution containing 0.9% NaCl in water, where the solution volume will be such that the drug concentration is after release is less than 5% of saturation. The mixture is maintained at 37°C and stirred slowly to maintain the implants in suspension. The appearance of the dissolved drug as a function of time may be followed by various methods known in the art, such as spectrophotometrically, HPLC, mass spectroscopy, etc. until the absorbance becomes constant or until greater than 90% of the drug has been released.

Additional pharmacologic or therapeutic agents which may find use in the present systems, include, without limitation, those disclosed in U.S. Pat. Nos. 4,474,451, columns 4-6 and 4,327,725, columns 7-8.

Examples of antihistamines include, and are not limited to, loradatine, hydroxyzine, diphenhydramine, chlorpheniramine, brompheniramine, cyproheptadine, terfenadine, clemastine, triprolidine, carbinoxamine, diphenylpyraline, phenindamine, azatadine, tripelennamine, dexchlorpheniramine, dexbrompheniramine, methdilazine, and trimprazine doxylamine, pheniramine, pyrilamine, chiorcyclizine, thonzylamine, and derivatives thereof.

Examples of antibiotics include without limitation, cefazolin, cephradine, cefaclor, cephapirin, ceftizoxime, cefoperazone, cefotetan, cefutoxime, cefotaxime, cefadroxil, ceftazidime, cephalexin, cephalothin,, cefamandole, cefoxitin, cefonicid, ceforanide, ceftriaxone, cefadroxil, cephradine, cefuroxime, ampicillin, amoxicillin, cyclacillin, ampicillin, penicillin G, penicillin V potassium, piperacillin, oxacillin, bacampicillin, cloxacillin, ticarcillin, azlocillin, carbenicillin, methicillin, nafcillin, erythromycin, tetracycline, doxycycline, minocycline, aztreonam, chloramphenicol, ciprofloxacin hydrochloride, clindamycin, metronidazole, gentamicin, lincomycin, tobramycin, vancomycin, polymyxin B sulfate, colistimethate, colistin, azithromycin, augmentin, sulfamethoxazole, trimethoprim, and derivatives thereof.

Examples of beta blockers include acebutolol, atenolol, labetalol, metoprolol, propranolol, timolol, and derivatives thereof.

Examples of other corticosteroids include cortisone, prednisolone, flurometholone, dexamethasone, medrysone, loteprednol, fluazacort, hydrocortisone, prednisone, betamethasone, prednisone, methylprednisolone, riamcinolone hexacatonide, paramethasone acetate, diflorasone, fluocinonide, derivatives thereof, and mixtures thereof.

Examples of antineoplastic agents include adriamycin, cyclophosphamide, actinomycin, bleomycin, duanorubicin, doxorubicin, epirubicin, mitomycin, methotrexate, fluorouracil, carboplatin, carmustine (BCNU), methyl-CCNU, cisplatin, etoposide, interferons, camptothecin and derivatives thereof, phenesterine, taxol and derivatives thereof, taxotere and derivatives thereof, vinblastine, vincristine, tamoxifen, etoposide, piposulfan, cyclophosphamide, and flutamide, and derivatives thereof.

Examples of immunosuppressive agents include cyclosporine, azathioprine, tacrolimus, and derivatives thereof.

Examples of antiviral agents include interferon gamma, zidovudine, amantadine hydrochloride, ribavirin, acyclovir, valciclovir, dideoxycytidine, phosphonoformic acid, ganciclovir, and derivatives thereof.

Examples of antioxidant agents include ascorbate, alpha-tocopherol, mannitol, reduced glutathione, various carotenoids, cysteine, uric acid, taurine, tyrosine, superoxide dismutase, lutein, zeaxanthin, cryotpxanthin, astazanthin, lycopene, N-acetyl-cysteine, carnosine, gamma-glutamylcysteine, quercitin, lactoferrin, dihydrolipoic acid, citrate, Ginkgo Biloba extract, tea catechins, bilberry extract, vitamins E or esters of vitamin E, retinyl palmitate, and derivatives thereof.

Other therapeutic agents include squalamine, carbonic anhydrase inhibitors, alpha agonists, prostamides, prostaglandins, antiparasitics, antifungals, and derivatives thereof.

In addition to therapeutic components, the intraocular implants disclosed herein may include effective amounts of buffering agents, preservatives and the like. Suitable water soluble buffering agents include, without limitation, alkali and alkaline earth carbonates, phosphates, bicarbonates, citrates, borates, acetates, succinates and the like, such as sodium phosphate, citrate, borate, acetate, bicarbonate, carbonate and the like. These agents advantageously present in amounts sufficient to maintain a pH of the system of between about 2 to about 9 and more preferably about 4 to about 8. As such the buffering agent may be as much as about 5% by weight of the total implant. Suitable water soluble preservatives include sodium bisulfite, sodium bisulfate, sodium thiosulfate, ascorbate, benzalkonium chloride, chlorobutanol, thimerosal, phenylmercuric acetate, phenylmercuric borate, phenylmercuric nitrate, parabens, methylparaben, polyvinyl alcohol, benzyl alcohol, phenylethanol and the like and mixtures thereof. These agents may be present in amounts of from 0.001 to about 5% by weight and preferably 0.01 to about 2% by weight.

In some situations mixtures of implants may be utilized employing the same or different pharmacological agents. In this way, a cocktail of release profiles, giving a biphasic or triphasic release with a single administration is achieved, where the pattern of release may be greatly varied.

Additionally, release modulators such as those described in U. S. Patent No. 5,869,079 may be included in the implants. The amount of release modulator employed will be dependent on the desired release profile, the activity of the modulator, and on the release profile of the glucocorticoid in the absence of modulator. Electrolytes such as sodium chloride and potassium chloride may also be included in the implant. Where the buffering agent or enhancer is hydrophilic, it may also act as a release accelerator. Hydrophilic additives act to increase the release rates through faster dissolution of the material surrounding the drug particles, which increases the surface area of the drug exposed, thereby increasing the rate of drug bioerosion. Similarly, a hydrophobic buffering agent or enhancer dissolve more slowly, slowing the exposure of drug particles, and thereby slowing the rate of drug bioerosion.

Various techniques may be employed to produce the implants described herein. Useful techniques include, but are not necessarily limited to, solvent evaporation methods, phase separation methods, interfacial methods, molding methods, injection molding methods, extrusion methods, co-extrusion methods, carver press method, die cutting methods, heat compression, combinations thereof and the like.

Specific methods are discussed in U.S. Pat. No. 4,997,652. Extrusion methods may be used to avoid the need for solvents in manufacturing. When using extrusion methods, the polymer and drug are chosen so as to be stable at the temperatures required for manufacturing, usually at least about 85 degrees Celsius. Extrusion methods use temperatures of about 25 degrees C to about 150 degrees C, more preferably about 65 degrees C to about 130 degrees C. An implant may be produced by bringing the temperature to about 60 degrees C to about 150 degrees C for drug/polymer mixing, such as about 130 degrees C, for a time period of about 0 to 1 hour, 0 to 30 minutes, or 5-15 minutes. For example, a time period may be about 10 minutes, preferably about 0 to 5 min. The implants are then extruded at a temperature of about 60 degrees C to about 130 degrees C, such as about 75 degrees C.

In addition, the implant may be coextruded so that a coating is formed over a core region during the manufacture of the implant.

Compression methods may be used to make the implants, and typically yield implants with faster release rates than extrusion methods. Compression methods may use pressures of about 50-150 psi, more preferably about 70-80 psi, even more preferably about 76 psi, and use temperatures of about 0 degrees C to about 115 degrees C, more preferably about 25 degrees C.

The implants of the present invention may be inserted into the eye, for example the vitreous chamber of the eye, by a variety of methods, including placement by forceps or by trocar following making a 2-3 mm incision in the sclera. The method of placement may influence the therapeutic component or drug release kinetics. For example, delivering the implant with a trocar may result in placement of the implant deeper within the vitreous than placement by forceps, which may result in the implant being closer to the edge of the vitreous. The location of the implant may influence the concentration gradients of therapeutic component or drug surrounding the element, and thus influence the release rates (e.g., an element placed closer to the edge of the vitreous may result in a slower release rate).

Among the diseases/conditions which can be treated or addressed in accordance with the present invention include, without limitation, the following:
MACULOPATHIES/RETINAL DEGENERATION: Non-Exudative Age Related Macular Degeneration (ARMD), Exudative Age Related Macular Degeneration (ARMD), Choroidal Neovascularization, Diabetic Retinopathy, Acute Macular Neuroretinopathy, Central Serous Chorioretinopathy, Cystoid Macular Edema, Diabetic Macular Edema.
UVEITIS/RETINITIS/CHOROIDITIS: Acute Multifocal Placoid Pigment Epitheliopathy, Behcet's Disease, Birdshot Retinochoroidopathy, Infectious (Syphilis, Lyme, Tuberculosis, Toxoplasmosis), Intermediate Uveitis (Pars Planitis), Multifocal Choroiditis, Multiple Evanescent White Dot Syndrome (MEWDS), Ocular Sarcoidosis, Posterior Scleritis, Serpignous Choroiditis, Subretinal Fibrosis and Uveitis Syndrome, Vogt-Koyanagi-Harada Syndrome.
VASCULAR DISEASES/EXUDATIVE DISEASES: Retinal Arterial Occlusive Disease, Central Retinal Vein Occlusion, Disseminated Intravascular Coagulopathy, Branch Retinal Vein Occlusion, Hypertensive Fundus Changes, Ocular Ischemic Syndrome, Retinal Arterial Microaneurysms, Coat's Disease, Parafoveal Telangiectasis, Hemi-Retinal Vein Occlusion, Papillophlebitis, Central Retinal Artery Occlusion, Branch Retinal Artery Occlusion, Carotid Artery Disease (CAD), Frosted Branch Angitis, Sickle Cell Retinopathy and other Hemoglobinopathies, Angioid Streaks, Familial Exudative Vitreoretinopathy, Eales Disease.
TRAUMATIC/SURGICAL: Sympathetic Ophthalmia, Uveitic Retinal Disease, Retinal Detachment, Trauma, Laser, PDT (photodynamic therapy), Photocoagulation, Hypoperfusion During Surgery, Radiation Retinopathy, Bone Marrow Transplant Retinopathy.
PROLIFERATIVE DISORDERS: Proliferative Vitreal Retinopathy and Epiretinal Membranes, Proliferative Diabetic Retinopathy.
INFECTIOUS DISORDERS: Ocular Histoplasmosis, Ocular Toxocariasis, Presumed Ocular Histoplasmosis Syndrome (POHS), Endophthalmitis, Toxoplasmosis, Retinal Diseases Associated with HIV Infection, Choroidal Disease Associated with HIV Infection, Uveitic Disease Associated with HIV Infection, Viral Retinitis, Acute Retinal Necrosis, Progressive Outer Retinal Necrosis, Fungal Retinal Diseases, Ocular Syphilis, Ocular Tuberculosis, Diffuse Unilateral Subacute Neuroretinitis, Myiasis.
GENETIC DISORDERS: Retinitis Pigmentosa, Systemic Disorders with Associated Retinal Dystrophies, Congenital Stationary Night Blindness, Cone Dystrophies, Stargardt's Disease and Fundus Flavimaculatus, Best's Disease, Pattern Dystrophy of the Retinal Pigmented Epithelium, X-Linked Retinoschisis, Sorsby's Fundus Dystrophy, Benign Concentric Maculopathy, Bietti's Crystalline Dystrophy, pseudoxanthoma elasticum.
RETINAL TEARS/HOLES: Retinal Detachment, Macular Hole, Giant Retinal Tear.
TUMORS: Retinal Disease Associated with Tumors, Congenital Hypertrophy of the RPE, Posterior Uveal Melanoma, Choroidal Hemangioma, Choroidal Osteoma, Choroidal Metastasis, Combined Hamartoma of the Retina and Retinal Pigmented Epithelium, Retinoblastoma, Vasoproliferative Tumors of the Ocular Fundus, Retinal Astrocytoma, Intraocular Lymphoid Tumors.
MISCELLANEOUS: Punctate Inner Choroidopathy, Acute Posterior Multifocal Placoid Pigment Epitheliopathy, Myopic Retinal Degeneration, Acute Retinal Pigment Epithelitis and the like.

As set forth, an embodiment of our invention is a treatment of an ocular condition (such as a posterior ocular condition) by placing into the vitreous of a patient's eye an implant which comprises an active agent, such as an anti-inflammatory compound, such as various steroids. The implant can also comprise a biodegradable, biocompatible polymer, such as a polylactic acid polyglycolic acid (PLGA) copolymer. After placement of the implant into the vitreous, PDT can be carried out - by introducing a photoactive agent into the eye (as by systemic administration to the patient of a suitable compound which can accumulate in certain ocular tissues) followed by irradiation of the eye so as to activate the photoactive agent.

Significantly, a therapy for CNV which uses a PDT in conjunction with (that is in combination with) use of an intravitreal implant which contains an anti-inflammatory active agent (such as a steroid) (i.e. a combination therapy) can provide a therapeutic result which is not achieved by separate use of either the PDT or the intravitreal, biodegradable, sustained release or extended release active agent implant to treat the patient's CNV. In other words, a synergy can be achieved by use of PDT and an intravitreal, biodegradable, sustained release or extended release active agent implant to treat the patient's CNV. The result is a more positive patient outcome (as measured by an improved, enhanced, repaired or retained patient visual acuity) due presumably to actions of the combination therapy on factors which contribute to treatment or alleviation of CNV.

Thus, at least the following synergies can occur through use of the combination therapy:
1. It is known that PDT can have toxic effects on the eye. For example, PDT while typically selectively damages newly formed and/or abnormal retinal blood vessels, can also result in damage healthy retinal and choroidal blood vessels. Additionally, PDT can cause an increase in factors which act to induce the growth of and leakage from areas of CNV. Thus, PDT can increase VEGF mediated CNV activity. Additionally, some patients treated with PDT have developed a rapid worsening of vision attributable to the PDT treatment itself. The combination therapy can reduce or eliminate these toxic effect of PDT on the eye, particularly when the active agent is a steroid, such as dexamethasone. For example, the toxic effects of PDT can be reduced by inhibiting or by repairing the damage caused by PDT to healthy retinal and choroidal blood vessels, and by reducing growth of an leakage from areas of CNV.
2. Use of the combination therapy to treat CNV can result in the synergy of more patients with an improved visual acuity, as compared to use of PDT alone or use alone of an intravitreal anti-inflammatory agent implant in the same patients
3. Typically PDT is administered about once every three months, for example to treat CNV. The combination therapy can reduce the required frequency of PDT treatment required to obtain an improved so that patients receiving the combination therapy can have PDT treatment less frequently that every 3 months and still obtain a visual acuity benefit equal to or superior to that obtained by the same or a similar patient upon use of PDT alone.
4. PDT typically benefits only a subset of CNV lesions, such as predominantly classic CNV, small minimally classic CNV, and small occult-only CNV. The combination therapy can be used to treat lesion compositions and sizes not effectively treated by PDT alone.

### Example 1

### Treatment of macular degeneration with a method of the present invention.

A 70 year old female patient complains of blind spots in her vision. Upon examination, the physician diagnoses her with the wet form of macular degeneration. Upon examination of her eyes, it is found that blood vessels have grown beneath the retina of each eye and are leaking blood and fluid which is causing the blind spots. On the day of scheduled treatment, an implant is surgically implanted into each one of her eyes, specifically into the vitreous of each eye, using a trocar and a 2 mm incision. Each of the implants comprises dexamethasone particles entrapped within a polylactic acid polyglycolic acid (PLGA) copolymer; more specifically each implant comprising about 70 percent by weight of dexamethasone and about 30 percent by weight of PLGA, wherein the total mass of the implant is about 1000 µg. Within a day of the surgery, an effective amount of verteporfin is administered to the patient by means of a slow intravenous infusion over a period of about 32 minutes. Photodynamic therapy is then performed on her eyes using a wavelength of light of about 689 nm or about 692 nm, with an irradiance of 600 mW/cm² and light exposure of 50J/cm². After two days, the patient is examined and there is found an absence of leakage at the back of the eyes. The implant is left to remain in the patient's eyes in order to provide continuous dosing of dexamethasone over the next two months. Vision is improved and further degeneration of vision is prevented.

### Example 2

### Treatment of Classic or Occult Subfoveal Choroidal Neovascularization due to Age-Related Macular Degeneration

A clinical study is carried out to treat patients with predominantly classic, minimally classic or occult subfoveal choroidal neovascularization due to age-related macular degeneration. The patients are treated with a combination therapy which comprises photodynamic therapy using Visudyne and intravitreal implantation of a one mg biodegradable PLGA implant which contains 700 µg of dexamethasone (Posurdex, Allergan, Inc., Irvine California). Further details regarding this dexamethasone intravitreal implant can be found in U.S. patent application serial number 10/837,357, filed April 30, 2004, where the implant is also referred to as a 700 µg DEX PS DDS. The placebo (sham) implant consists of about one mg of the same PLGA copolymer, with any dexamethasone being present.

Visudyne (verteporfin for injection) (Novartis Pharmaceuticals, East Hanover, New Jersey) is a light-activated therapy indicated for the treatment of patients with predominantly classic subfoveal choroidal neovascularization (CNV) secondary to wet AMD, as well as pathologic myopia (PM) and ocular histoplasmosis syndrome (OHS). Visudyne apparently acts through a photothrombic effect on blood vessels and is used to help preserve visual acuity and slow or stop the advancement of CNV. Visudyne utilizes a lipophilic molecule (known as verteporfin) to occlude abnormal blood vessels found in the eye while generally sparing overlying retinal tissue. We use Visudyne according to the TAP protocol¹. Generally, Visudyne is used as a multicourse therapy with patients receiving it once every 3 months as long as leakage appears on fluorescein angiography. Visudyne therapy is a 2-stage
¹Schmidt-Erfurth U, Hasan T. Mechanisms of action of photodynamic therapy with verteporfin for the treatment of age-related macular degeneration. Surv Ophthalmol. 2000;45:195-214; Treatment of Age-Related Macular Degeneration With Photodynamic Therapy (TAP) Study Group. Verteporfin therapy of subfoveal choroidal neovascularization in patients with age-related macular degeneration: additional information regarding baseline lesion composition's impact on vision outcomes-TAP report no. 3. Arch Ophthalmol. 2002;120:1443-1454; Treatment of Age-Related Macular Degeneration With Photodynamic Therapy (TAP) Study Group. Photodynamic therapy of subfoveal choroidal neovascularization in age-related macular degeneration: one-year results of 2 randomized clinical trials-TAP report 1. Arch Ophthalmol. 1999;117:1329-1345, and; Treatment of Age-Related Macular Degeneration With Photodynamic Therapy (TAP) Study Group. Photodynamic therapy of subfoveal choroidal neovascularization in age-related macular degeneration with verteporfin: two-year results of 2 randomized clinical trials-TAP report no. 2. Arch Ophthalmol. 2001;119:198-207.

process requiring both the intravenous administration of verteporfin and the application of non-thermal red light. Upon injection, verteporfin is transported in the plasma primarily by lipoproteins. The drug is then activated by non-thermal light, which when applied in the presence of oxygen, results in the creation of highly reactive, short-lived singlet oxygen and oxygen free radicals. These molecules in turn cause selective damage to the neovascular endothelium, resulting in vessel occlusion. Damaged endothelial tissue is known to release procoagulant and vasoactive factors through the lipo-oxygenase (leukotriene) and cyclo-oxygenase (eicosanoids such as thromboxane) pathways, resulting in platelet aggregation, fibrin clot formation, and vasoconstriction.

The objective of this clinical study is to evaluate the safety and efficacy of the 700µg dexamethasone implant in combination with photodynamic therapy (compared with PDT using the same pars plana approach to the vitreous), to treat patients with predominantly classic, minimally classic or occult subfoveal neovascularization due to age-related macular degeneration. The implants are inserted into the vitreous using the applicator set forth in U.S. patent application serial number 11/021,947, filed December 23, 2004.

Patients are allocated in a 1:1 ratio (700µg dexamethasone implant plus PDT: PDT) on the randomization (day 0) visit and are followed for 24 months. Each patient has at least one with a diagnosis of classic, or active minimally classic or active occult (with no classic) subfoveal CNV due to AMD (age related macular degeneration).

The primary efficacy measure is the proportion of patients experiencing an improvement of 15 letters or more from baseline of BCVA using the ETDRS method. The secondary efficacy measures include: the proportion of patients experiencing a loss of 15 letters or more from baseline of BCVA using the ETDRS method; change from baseline in BCVA; change from baseline (based on fluorescein angiography) in total lesion area, CNV lesion area, CNV/total lesion area, and area of fluorescein leakage; maximal retinal thickness in any central subfield by Optical Coherence Tomography (OCT); and the number of PDT treatments required

It is determined that the 700µg dexamethasone implant in conjunction with photodynamic therapy is more effective than use of a placebo implant with photodynamic therapy in improving best-corrected visual acuity (BCVA) (as measured by the proportion of patients experiencing at least a 15-letter increase from baseline in the study eye using the Early Treatment Diabetic Retinopathy Study (ETDRS) method). The 700 µg dexamethasone implant in conjunction with PDT is therefore effective to treat macular degeneration.

While this invention has been described with respect to various specific examples and embodiments, it is to be understood that the invention is not limited thereto and that it can be variously practiced within the scope of the following claims.

## Claims

1. Use of a combination of (i) a bioerodible implant that comprises an anti-inflammatory component and a bioerodible polymeric component, and (ii) a photoactive agent for the manufacture of a medicament for the treatment of an ocular condition.

2. The use of claim 1, wherein the photoactive agent comprises an agent selected from the group consisting of porphyrin, verteporfin, hematoporphyrins, hematoporphyrin derivatives, pheophorbides, derivates of pheophorbides, bacteriochlorins, purpurins, merocyanines, porphycenes, and combinations thereof.

3. The use of claim 1, wherein the anti-inflammatory component comprises a steroidal anti-inflammatory agent.

4. The use of claim 1, wherein the anti-inflammatory component is selected from the group consisting of cortisone, dexamethasone, fluocinolone, hydrocortisone, methylprednisolone, prednisolone, prednisone, triamcinolone, derivatives thereof, and mixtures thereof.

5. The use of claim 1, wherein the anti-inflammatory component is selected from the group consisting of corticosteroids and mixtures thereof.

6. The use of claim 1, wherein the anti-inflammatory component is dexamethasone.

7. The use of claim 1, wherein the bioerodible implant further comprises an ophthalmically acceptable therapeutic agent in addition to the anti-inflammatory component.

8. The use of claim 1, wherein the bioerodible polymeric component comprises a polymeric material selected from the group consisting of polymers of poly-lactic acid, polymers of poly-glycolic acid, copolymers of lactic acid and glycolic acid, and mixtures thereof.

9. The use of claim 1, wherein the bioerodible polymeric component comprises a polymeric material selected from the group consisting of copolymers of lactic acid and glycolic acid, and mixtures thereof.

10. The use of claim 1, wherein the bioerodible implant comprises a polylactic acid polyglycolic acid copolymer (PLGA) and dexamethasone.

11. The use of claim 1, wherein the ocular condition is **characterized by** retinal abnormalities.

12. The use of claim 1, wherein the ocular condition is macular degeneration or subfoveal neovascularization.

13. The use of claim 1, wherein the bioerodible implant is an intravitreal implant.

14. The use of claim 1, wherein the photoactive agent is provided in a composition for intravenous administration.

15. The use of claim 1, wherein the photoactive agent is activated by laser irradiation.

## Patentansprüche

1. Verwendung einer Kombination eines bioerodierbaren Implantats (i), das eine entzündungshemmende Komponente und eine bioerodierbare Polymerkomponente umfasst, und eines fotoaktiven Mittels (ii) zur Herstellung eines Medikaments zur Behandlung eines Augenzustands.

2. Verwendung gemäss Anspruch 1, worin das fotoaktive Mittel ein Mittel umfasst, das ausgewählt ist aus der Gruppe bestehend aus Porphyrin, Verteporfin, Hämatoporphyrinen, Hämatoporphyrinderivaten, Pheophorbiden, Derivaten von Pheophorbiden, Bakteriochlorinen, Purpurinen, Merocyaninen, Porphycenen und Kombinationen davon.

3. Verwendung gemäss Anspruch 1, worin die entzündungshemmende Komponente ein steroidales entzündungshemmendes Mittel umfasst.

4. Verwendung gemäss Anspruch 1, worin die entzündungshemmende Komponente ausgewählt ist aus der Gruppe bestehend aus Kortison, Dexamethason, Fluocinolon, Hydrocortison, Methylprednisolon, Prednisolon, Prednison, Triamcinolon, Derivaten davon und Mischungen davon.

5. Verwendung gemäss Anspruch 1, worin die entzündungshemmende Komponente ausgewählt ist aus der Gruppe bestehend aus Corticosteroiden und Mischungen davon.

6. Verwendung gemäss Anspruch 1, worin die entzündungshemmende Komponente Dexamethason ist.

7. Verwendung gemäss Anspruch 1, worin das bioerodierbare Implantat zusätzlich zu der entzündungshemmenden Komponente ein ophthalmisch annehmbares therapeutisches Mittel umfasst.

8. Verwendung gemäss Anspruch 1, worin die bioerodierbare Polymerkomponente ein Polymermaterial umfasst, das ausgewählt ist aus der Gruppe bestehend aus Polymeren der Polymilchsäure, Polymeren der Polyglykolsäure, Copolymeren von Milchsäure und Glykolsäure und Mischungen davon.

9. Verwendung gemäss Anspruch 1, worin die bioerodierbare Polymerkomponente ein Polymermaterial umfasst, das ausgewählt ist aus der Gruppe bestehend aus Copolymeren von Milchsäure und Glykolsäure und Mischungen davon.

10. Verwendung gemäss Anspruch 1, worin das bioerodierbare Implantat ein Polymilchsäure-Polyglykolsäure-Copolymer (PLGA) und Dexamethason umfasst.

11. Verwendung gemäss Anspruch 1, worin der Augenzustand durch retinale Anomalien **gekennzeichnet** ist.

12. Verwendung gemäss Anspruch 1, worin der Augenzustand Makuladegeneration oder subfoveale Neovaskularisation ist.

13. Verwendung gemäss Anspruch 1, worin das bioerodierbare Implantat ein intravitreales Implantat ist.

14. Verwendung gemäss Anspruch 1, worin das fotoaktive Mittel in einer Zusammensetzung für eine intravenöse Verabreichung bereitgestellt wird.

15. Verwendung gemäss Anspruch 1, worin das fotoaktive Mittel durch Laserstrahlen aktiviert wird.

## Revendications

1. Utilisation d'une combinaison de (i) un implant bio-érodable comprenant un composant anti-inflammatoire et un composant polymère bio-érodable et (ii) un agent photoactif pour la fabrication d'un médicament pour le traitement d'une condition oculaire.

2. Utilisation selon la revendication 1, dans laquelle l'agent photoactif comprend un agent choisi dans le groupe constitué de porphyrine, de vertéporfine, d'hématoporphyrines, de dérivés d'hématoporphyrine, de phéophorbides, de dérivés de phéophorbides, de bactériochlorines, de purpurines, de mérocyanines, de porphycènes, et de combinaisons de ceux-ci.

3. Utilisation selon la revendication 1, dans laquelle le composant anti-inflammatoire comprend un agent anti-inflammatoire stéroïdien.

4. Utilisation selon la revendication 1, dans laquelle le composant anti-inflammatoire est choisi dans le groupe constitué de cortisone, de dexaméthasone, de fluocinolone, d'hydrocortisone, de méthylprednisolone, de prednisolone, de prednisone, de triamcinolone, de dérivés et de mélanges de ceux-ci.

5. Utilisation selon la revendication 1, dans laquelle le composant anti-inflammatoire est choisi dans le groupe constitué de corticostéroïdes et de mélanges de ceux-ci.

6. Utilisation selon la revendication 1, dans laquelle le composant anti-inflammatoire est la déxaméthasone.

7. Utilisation selon la revendication 1, dans laquelle l'implant bio-érodable comprend en outre un agent thérapeutique ophtalmiquement acceptable en plus du composant anti-inflammatoire. '

8. Utilisation selon la revendication 1, dans laquelle le composant polymère bio-érodable comprend un matériau polymère choisi dans le groupe constitué de polymères d'acide polylactique, de polymères d'acide polyglycolique, de copolymères d'acide lactique et d'acide glycolique et de mélanges de ceux-ci.

9. Utilisation selon la revendication 1, dans laquelle le composant polymère bio-érodable comprend un matériau polymère choisi dans le groupe constitué de copolymères d'acide lactique et d'acide glycolique et de mélanges de ceux-ci.

10. Utilisation selon la revendication 1, dans laquelle l'implant bio-érodable comprend un copolymère d'acide polylactique-acide polyglycolique (PLGA) et de la dexaméthasone.

11. Utilisation selon la revendication 1, dans laquelle la condition oculaire est **caractérisée par** des anomalies rétiniennes.

12. Utilisation selon la revendication 1, dans laquelle la condition oculaire est une dégénérescence maculaire ou une néo-vascularisation subfovéale.

13. Utilisation selon la revendication 1, dans laquelle l'implant bio-érodable est un implant intra-vitréen.

14. Utilisation selon la revendication 1, dans laquelle l'agent photoactif est fourni dans une composition pour administration par voie intraveineuse.

15. Utilisation selon la revendication 1, dans laquelle l'agent photoactif est activé par rayonnement laser.
